# EUROPEAN PATENT APPLICATION

(11) **EP 3 231 419 A1**
(43) Date of publication of application: **18.10.2017**
(21) Application number: 16165388.6
(22) Date of filing: 14.04.2016
(51) Int. Cl.: A61K 9/16, C12N 15/10

(54) **GENERATION OF DNA HYDROGELS FROM LINEAR BUILDING BLOCKS**

(71) Applicant: Universität Siegen, 57068 Siegen (DE)
(72) Inventor: Nöll, Tanja, 57482 Wenden-Heid (DE); Nöll, Gilbert, 57482 Wenden-Heid (DE)
(74) Representative: Fuchs Patentanwälte Partnerschaft mbB

(57) **Abstract**

The present invention relates to a method for producing a DNA hydrogel as well as a DNA hydrogel obtained by the method and the use of such DNA hydrogel.

## Description

DNA hydrogels are known from the prior art.

Several groups have described formation of DNA hydrogels by cross-linking DNA molecules with chemical cross-linkers as for example ethylene glycol diglycidyl ether (EGDE). However, such DNA hydrogels have several disadvantages, especially in healthcare applications. On the one hand, the chemical cross-linker will be present in such DNA hydrogels and thus in the final products. Presence of the chemical cross-linkers might be associated with allergic reactions or other unwanted side effects. On the other hand, DNA hydrogels that have been obtained by use of chemical cross-linker consist of permanently cross-linked polymers that cannot be depolymerized. However, depolymerization of the polymers might be desired, particular in applications that comprise controlled drug-release. Moreover, biodegradability of chemically cross-linked DNA hydrogels is very poor. Furthermore, chemically cross-linked DNA hydrogels have also structural disadvantages. Particularly, chemical cross-linkers are generally hydrophobic molecules that may agglomerate in hydrophilic environment. Therefore, chemical cross-linkers tend to be present in local clusters of high concentration. The cross-linkers are not homogeneously distributed throughout the DNA hydrogel. Consequently, as the degree of cross-linking is strongly dependent on the concentration of cross-linker, the degree of cross-linking is very heterogeneous throughout the DNA hydrogel. However, heterogeneous materials are problematic in most applications and therefore more homogeneous DNA hydrogels are desirable.

Thus, there have been several attempts to create pristine DNA hydrogels, which are highly biocompatible and biodegradable. Pristine DNA hydrogels in the sense of the present invention are hydrogels that do not comprise any chemical cross-linker. Pristine DNA hydrogels may, however, comprise other components in minor amounts. For example, pristine DNA hydrogels may comprise residuals of DNA ligases without any negative influence on the performance of the gel.

LaBean (Nature Materials (2006), pages 767 to 768) and Um et al. (Nature Materials (2006), pages 797 to 801) describe pristine DNA hydrogels that were formed by self-assembly of flexible branched DNA building blocks equipped with palindromic sticky ends. Self-assembly by hybridization was followed by enzymatic ligation, which resulted in the formation of covalent bonds between the individual building blocks. These hydrogels were evaluated for applications as drug capture and release systems. It turned out that not only the enzyme-catalyzed gel formation but also the release of entrapped molecules from this type of DNA hydrogel was rather slow.

Cheng et al. (Angewandte Chemie International Edition (2009), pages 7660 to 7663) disclosed a fast responding pH-triggered DNA hydrogel, which has been developed from Y-shaped DNA building blocks equipped with cytosine-rich interlocking i-motif domains. Xing et al. (Advanced Materials (2011), pages 1117 to 1121) disclosed DNA hydrogels formed by hybridization of Y-DNA building blocks connected via linear double-strand DNA (dsDNA) that served as a linker.

Recently, Nöll et al. (Angewandte Chemie International Edition (2014), pages 8328 to 8332) disclosed DNA hydrogels made from short linear dsDNA building blocks comprising single-strand DNA (ssDNA) overhangs (sticky ends). These hydrogels are formed solely by hybridization and are thus thermo-responsive.

Usage of those prior art DNA hydrogels is limited by the rather high costs and the restricted availability of the building blocks used for DNA hydrogel formation. These building blocks are DNA oligomers with specific sequences, which have to be prepared by chemical solid phase synthesis. This is very expensive. Therefore, the biomedical industry is currently refraining from exploring the possible advantages of DNA hydrogels. If DNA hydrogels were made available at larger scale and with reasonable costs, this might pave the way for implementation of DNA hydrogels in various applications such as cell transplant therapy, bio-mineralization, tissue engineering, gene therapy, drug release and bio-sensing.

Furthermore, it has to be taken into account that the length of such chemically synthesized DNA building blocks is limited because the efficiency of chemical solid phase synthesis is substantially below 100% so that price and effort for obtaining longer oligomers is increasing exponentially with the desired number of base pairs. However, longer building blocks might be desired for specifically adjusting properties of the DNA hydrogels. Particularly, the viscoelastic properties may depend strongly on the length of the building blocks.

It is therefore an object of the present invention to overcome the problems of the prior art and to provide a method for producing DNA hydrogels that enables large scale production of DNA hydrogels with specifically adjusted properties at reduced costs.

The problem is solved by the subject-matter of the patent claims.

The problem is particularly solved by a method for producing a DNA hydrogel comprising the following steps:
a) Providing basis DNA
b) Enzymatically digesting basis DNA to obtain linear dsDNA building blocks, and
c) Providing DNA hydrogel by assembly of the building blocks, wherein the assembly involves DNA hybridization and/or enzymatic ligation.

Basis DNA is provided according to step a) of the method of the present invention. The basis DNA may be any DNA that is available at large scale. Preferably, the basis DNA is based on DNA from biological or biotechnological sources. Preferably, the basis DNA may be selected from the group consisting of Herring Sperm DNA, Salmon Sperm DNA, lambda phage DNA and plasmid DNA. Most preferably, the basis DNA is plasmid DNA.

Plasmid DNA is advantageous because it is well characterized with regard to the nucleotide sequence and the sequence can also easily be manipulated by genetic means. Targeted manipulation of the sequence of the basis DNA may facilitate specific adjustment of the properties of the resulting DNA hydrogel. Fragments with undesired DNA sequence obtained upon enzymatic restriction digest of plasmid DNA, for example sequences harboring antibiotic resistance genes, may easily be separated from the desired dsDNA building blocks so that DNA hydrogels comprising only building blocks of known and desired DNA sequence can be obtained. Such separation may for example be achieved dependent on the size of the fragments by gel electrophoresis and subsequent purification of the desired dsDNA building blocks from the gel.

Furthermore, another advantage of plasmid DNA is that plasmid DNA is available at extraordinary large scale by fermentation. Moreover, plasmid DNA may be manipulated such that it contains repetitive elements that allow obtaining several dsDNA building blocks of identical sequence from a single copy of the plasmid upon enzymatic digest. Such repeats preferably comprise the sequence of the desired dsDNA building blocks as well as suitable flanking sequences that allow the targeted enzymatic digest. In preferred embodiments, plasmid DNA of the present invention comprises at least 2, more preferably at least 5, more preferably at least 10, more preferably at least 20, more preferably at least 50, more preferably at least 100, more preferably at least 200, more preferably at least 500, more preferably at least 1000 repeats that allow obtaining an equal number of dsDNA building blocks of identical sequence from a single copy of the plasmid upon enzymatic digest. Preferably, basis DNA is provided in cyclic form. In an alternative embodiment, plasmid DNA may be linearized by enzymatic digest occurring at only one site in the plasmid. The obtained linearized plasmid DNA may be assembled into oligomers and/or entangled rings by enzymatic ligation. In such embodiment, repetitive elements in the plasmid DNA are not necessary and also DNA purification does not have to be performed after restriction digest. However, the restriction enzyme has to be deactivated after restriction digest.

Typically, plasmid DNA comprises a few thousand base pairs (bp). Preferable plasmid DNA comprises at least 1000 bp, more preferably at least 2000 bp, more preferably at least 3000 bp, more preferably at least 4000 bp, more preferably at least 5000 bp in order to enable efficient fermentative amplification of the plasmid DNA. However, the size of the plasmid DNA should also not be too high because efficiency of fermentative amplification might drop. Therefore, plasmid DNA preferably comprises at most 50000 bp, more preferably at most 30000 bp, more preferably at most 20000 bp, more preferably at most 15000 bp, more preferably at most 10000 bp. In preferred embodiments, plasmid DNA is present in form of high copy plasmids in order to obtain a large number of plasmids per cell.

Linear dsDNA building blocks are obtained by enzymatic digest of the basis DNA according to step b) of the method of the present invention.

Preferably, the enzymatic digest is done with at least one restriction enzyme. Restriction enzymes enable digest of basis DNA at enzyme-specific restriction sites so that linear dsDNA building blocks of defined size can be obtained. More preferably, enzymatic digest is done with at least two restriction enzymes, in particular exactly two restriction enzymes. Digest with more than one restriction enzyme may result in building blocks with two distinct ends. A single building block with two distinct ends cannot connect head-to-tail to itself. However, several of such building blocks may assemble into oligomers or polymers by inter-building-block head-to-head and tail-to-tail connection. Preferably, enzymatic digest is done with at most five, more preferably at most four, more preferably at most three restriction enzymes. A high number of restriction enzymes may reduce the efficiency of the digest.

Preferably, the linear dsDNA building blocks have a length of at least 10 bp, more preferably at least 50, more preferably at least 100 bp, more preferably at least 200 bp, more preferably at least 500 bp, more preferably at least 1000 bp. The present inventors found that the viscosity of the resulting hydrogels may depend on the length of the employed dsDNA building blocks. Namely, when the same total amount of DNA is used, shorter dsDNA building blocks result in relatively stiff DNA hydrogels having a comparably low entanglement length and pore size, while longer dsDNA building blocks result in softer DNA hydrogels having a comparably high entanglement length and pore size. The ability to adjust the viscoelastic properties is advantageous in various applications of DNA hydrogels. However, the dsDNA building blocks should also not be very long because assembly might be negatively affected. Therefore, the linear dsDNA building blocks preferably have a length of at most 20000 bp, more preferably at most 10000 bp, more preferably at most 5000 bp, more preferably at most 4000 bp, more preferably at most 3000 bp. By suitable selection of the dsDNA building blocks, the properties of the DNA hydrogels can be specifically adjusted to the desired use. In particular, mesh width, entanglement length and viscosity and more generally the viscoelastic properties of the DNA hydrogels of the present invention can be specifically adjusted in wide ranges.

Preferably, the linear dsDNA building blocks obtained by the enzymatic digest comprise at least one ssDNA overhang (sticky end). More preferably, the linear dsDNA building blocks comprise two ssDNA overhangs. Such overhangs may facilitate assembly of the building blocks by specific hybridization of the overhangs of distinct building blocks. The overhang comprises at least 1, more preferably at least 2, more preferably at least 3, more preferably at least 4, more preferably at least 5, more preferably at least 6, more preferably at least 8, more preferably at least 10, more preferably at least 15, more preferably at least 20 nucleotides. The more nucleotides the overhang comprises, the better is the specific hybridization of corresponding overhangs. However, the overhang should not comprise a very high number of nucleotides. Otherwise, binding of the overhang either to itself or to other unspecific nucleotides may occur. For example, by self-hybridization the overhang may form hairpins or other undesired structures that may prevent specific hybridization of corresponding overhangs. Therefore, the overhang preferably comprises at most 100, more preferably at most 50, more preferably at most 30 nucleotides. In alternative embodiments, the linear dsDNA building blocks do not comprise ssDNA overhangs. Thus, such building blocks do not have sticky ends but blunt ends.

Depending on the nucleotide sequence of the basis DNA and on the enzymes used for digest of the basis DNA, linear dsDNA building blocks of different lengths may be obtained in a single digest reaction. This can be achieved using a single restriction enzyme in case several restriction sites having a sequence suitable for digest with the respective restriction enzyme are distributed throughout the basis DNA. Alternatively, longer and shorter dsDNA building blocks may be obtained by simultaneous or consecutive digest with different restriction enzymes. In any case, the distance between the restriction sites that are recognized by the applied restriction enzyme(s) determines the size of the obtained dsDNA building blocks. Depending on the desired properties of the dsDNA hydrogel, dsDNA building blocks may be assembled such that the assembly is formed from dsDNA building blocks of identical length (homogeneous assembly) or from dsDNA building blocks of different lengths (heterogeneous assembly). A homogeneous assembly is obtained if the linear dsDNA building blocks obtained by digest of the basis DNA are homogeneous with regard to their length or if the obtained linear dsDNA building blocks are separated according to their length prior to assembly or if obtained linear dsDNA building blocks of different size harbor non-complementary ssDNA overhangs that prevent assembly of dsDNA building blocks of different length and foster assembly of dsDNA building blocks of homogeneous length by complementarity of their ssDNA overhangs. In the last case, simultaneous assembly of large dsDNA building blocks into a network with a comparably high entanglement length and pore size and of shorter dsDNA building blocks into a network with a comparably low entanglement length and pore size may be accompanied by formation of a super-assembly of both networks interweaved with each other.

In one embodiment of the present invention, a DNA hydrogel is provided by assembly of building blocks of different lengths without separation of the building blocks according to their length. Assembly of such a mixture of shorter and longer dsDNA building blocks may result in DNA hydrogels that comprise more than one assembly network, for example a double-network. Such DNA hydrogels may have improved mechanical properties. In alternative embodiments, the method of the present invention further comprises the step of separating the obtained building blocks according to their length prior to assembly. This facilitates specific adjustment of the properties of the DNA hydrogels by selection of defined and homogeneous building blocks.

In particular embodiments, basis DNA containing exactly one restriction site for a specific restriction enzyme is digested with the respective enzyme as the only restriction enzyme. In such embodiments building blocks consisting of linearized full-length basis DNA are obtained. Assembly of such building blocks may occur in form of concatenation, which might lead to DNA hydrogels with advantageous properties depending on the basis DNA.

The method of the present invention may further comprise the step of adding non-linear dsDNA building blocks prior to assembly. Non-linear dsDNA building blocks may also be termed branched dsDNA building blocks. Preferably, non-linear dsDNA building blocks have up to 10, more preferably up to 8, more preferably up to 6, more preferably up to 5, more preferably up to 4 connectable ends. Preferred non-linear dsDNA building blocks are selected from the group consisting of star-shaped dsDNA building blocks, T-shaped dsDNA building blocks, X-shaped dsDNA building blocks and Y-shaped dsDNA building blocks. T-shaped dsDNA building blocks and Y-shaped dsDNA building blocks have 3 connectable ends. X-shaped dsDNA building blocks have 4 connectable ends. Star-shaped dsDNA building blocks may have 5, 6, 7, 8, 9 or even 10 connectable ends. X-shaped dsDNA building blocks are most preferred. Preferred X-shaped dsDNA building blocks can be enzymatically obtained from linear dsDNA by use of recombinase enzymes. Such enzymes catalyze formation of X-shaped dsDNA building blocks in form of so-called Holliday junctions.

In preferred embodiments, non-linear dsDNA building blocks have ssDNA overhangs. Such ssDNA overhangs may be advantageous for establishing specific base-pair interactions of the non-linear dsDNA building blocks with the linear dsDNA building blocks obtained by enzymatic digest of basis DNA. In alternative embodiments, the non-linear dsDNA building blocks do not have ssDNA overhangs.

Non-linear dsDNA building may be added for specifically adjusting the properties of the DNA hydrogel resulting from co-assembly of linear and non-linear building blocks. Non-linear dsDNA building blocks are preferably added in an amount of at least 0.1 wt.-%, more preferably at least 0.2 wt.-%, more preferably at least 0.5 wt.-% based on the weight of the linear dsDNA building blocks. However, non-linear building blocks are very expensive and not available in very large scale. Furthermore, improvement of DNA hydrogel properties is not correlated with very high amounts non-linear dsDNA building blocks. Therefore, non-linear dsDNA building blocks are preferably added in an amount of at most 10 wt.-%, more preferably at most 5 wt.-%, more preferably at most 2 wt.-% based on the weight of the linear dsDNA building blocks.

A DNA hydrogel is provided by assembly of the building blocks, wherein the assembly involves DNA hybridization and/or enzymatic ligation according to step c) of the method of the present invention. Thus, formation of the hydrogel is based on assembly of the building blocks. In contrast to the monomeric building blocks that are dissolved in the surrounding assembly medium, assembled building blocks of the present invention provide gel-like rheological properties.

According to the present invention, assembly may be oligomerization, polymerization and/or concatenation. Assembly may also be self-assembly.

Unless the linear dsDNA building blocks are entirely palindromic, the building blocks will have two distinct ends that are termed "head" and "tail", respectively, in the present description. Assembly of the building blocks may occur by head-to-head, tail-to-tail and/or head-to-tail connection of the building blocks.

Preferably, the assembly medium is an aqueous salt-containing solution. Preferably, the amount of salt in the assembly medium is at least 50 mM, more preferably at least 100 mM, more preferably at least 150 mM, more preferably at least 200 mM, more preferably at least 300 mM. If the amount of salt in the assembly medium is very low, unspecific interactions of dsDNA building blocks may occur, which may deteriorate the desired controlled assembly of the building blocks and may thus deteriorate or even prevent efficient provision of DNA hydrogels. However, the amount of salt in the assembly medium should also not be very high because otherwise the specific assembly of the dsDNA building blocks may be deteriorated or even prevented. Therefore, the amount of salt in the assembly medium is preferably at most 2.0 M, more preferably at most 1.5 M, more preferably at most 1.0 M, more preferably at most 750 mM, more preferably at most 500 mM. A preferred salt is NaCl. The assembly medium preferably additionally comprises M²⁺ ions. Preferably, the concentration of the M2+ ions in the assembly medium is from 0.1 to 100 mM, more preferably from 0.5 to 30 mM, more preferably from 2 to 10 mM, even more preferably from 4 to 6 mM. M²⁺ ions present in such amounts may facilitate assembly of dsDNA building blocks. Preferably, M is selected from the group consisting of Be, Mg and Ca. Most preferably, M is Mg.

Preferably, the pH value of the assembly medium is in the range of between 5 and 8. Such pH values allow for efficient assembly of the dsDNA building blocks. Preferably, the pH value of the assembly medium is at least 5.5, more preferably at least 6.0, more preferably at least 6.5, more preferably at least 7.0. Preferably, the pH value of the assembly medium is at most 7.5. In a preferred embodiment, the pH value of the assembly medium is about 7.4.

Above indicated properties of the assembly medium with regard to salt and ion concentrations as well as pH values are advantageous for the hybridization reaction. In case assembly involves enzymatic ligation in addition to or instead of hybridization, the assembly medium has to be adapted to the requirements of the applied restriction enzyme(s) with regard to salt concentrations and pH. Additionally, in such a case the assembly medium has to contain adenosine triphosphate (ATP) in sufficient amounts.

Particularly, provision of a DNA hydrogel is successful according to the present invention once the storage modulus G' is at least as high as the loss modulus G". The degree of assembly that corresponds to storage modulus and loss modulus being equal is known to the skilled person as the gel point. Storage modulus and loss modulus can be measured with a suitable rheometer. Rheometers are well known to the skilled person. Preferred rheometers are Kinexus Ultra by Malvern as well as the Discovery Hybrid Rheometer DHR-2 by TA Instruments. Such rheometers are oscillation rheometers and comprise one plate for the sample and an opposite cone plate (alternative geometries for measurements can be applied as well). The restoring force is measured while one part of the geometry oscillates. Amplitude and frequency of the oscillation of the geometry can be regulated by the experimenter. Typically, amplitude of oscillation is chosen to be in between 0.1 % and 5% of the distance between sample plate and cone plate (if a cone plate geometry is used). Frequency is typically chosen to be in between 0.05 and 100 Hz. Also alternative measurement modes are possible. Instead of shear strain controlled also shear stress controlled measurements can be carried out.

In embodiments, in which the linear dsDNA building blocks comprise ssDNA overhangs, the assembly of the building blocks preferably involves hybridization of the overhangs. This enables specific control of association of the building blocks depending on the respective overhangs by appropriate selection of restriction enzymes that produce suitable ssDNA overhangs. The stability of such assemblies obtained by hybridization of corresponding overhangs depends on the length and on the nucleotide sequence of the overhangs. Longer overhangs as well as GC-rich overhangs will result in more stable association of the building blocks and thus more stable assemblies if they are formed by hybridization. Disassembly may occur at elevated temperatures and/or low concentrations. As disassembly results in dissolution of the DNA hydrogel, a DNA hydrogel comprising such loosely associated building blocks is especially suited for drug-delivery applications because drug-release can easily be initiated by disassembly.

It may be advantageous to further assist hybridization-induced assembly of the building blocks by enzymatic ligation. Thus, assembly of the building blocks may involve both hybridization and enzymatic ligation. Enzymatic ligation is particularly preferred in embodiments, in which the ssDNA overhangs are short. Enzymatic ligation results in covalent linkage of the building blocks and thereby prevents disassembly at elevated temperatures and/or low concentrations. However, as such gels are not permanently cross-linked with a chemical cross-linker, disassembly may be achieved enzymatically by nucleases. As suitable nucleases are present in the human or animal body, also DNA hydrogels comprising building blocks that have been covalently linked by enzymatic ligation may be used in drug delivery applications. Enzymatic ligation is preferably done with a DNA ligase. Preferable DNA ligases are selected from the group consisting of *E*. *coli* DNA ligase and T4 DNA ligase. More preferably, the DNA ligase is T4 DNA ligase because T4 DNA ligase may function in absence of NAD.

In embodiments in which the linear dsDNA building blocks do not comprise ssDNA overhangs, assembly of the building blocks is preferably achieved by enzymatic ligation as such dsDNA building blocks lack ssDNA overhangs for hybridization. Enzymatic ligation is preferably done with a DNA ligase. Preferable DNA ligases are selected from the group consisting of *E*. *coli* DNA ligase and T4 DNA ligase. T4 DNA ligase is most preferred in such embodiments because T4 DNA ligase has a substantially larger efficiency in ligating blunt end DNA as compared to *E*. *coli* DNA ligase.

According to the present invention is also DNA hydrogel obtained by the method of the present invention comprising assembled dsDNA building blocks and the surrounding liquid medium. More preferably, assembled dsDNA building blocks and liquid medium constitute at least 90 wt.-%, more preferably at least 95 wt.-%, more preferably at least 98 wt.-%, more preferably at least 99 wt.-%, more preferably at least 99.9 wt.-% of the total weight of the DNA hydrogel. Preferably, the liquid medium is selected from the group consisting of water and aqueous buffer solutions. A preferable aqueous buffer solution is the assembly medium described above.

According to the present invention is also the use of a DNA hydrogel of the present invention in applications in cell transplant therapy, bio-mineralization, tissue engineering, gene therapy, drug delivery, wound healing, cell culture, cell-free protein biosynthesis, bio-sensing, cosmetics or 3D printing.

### Examples

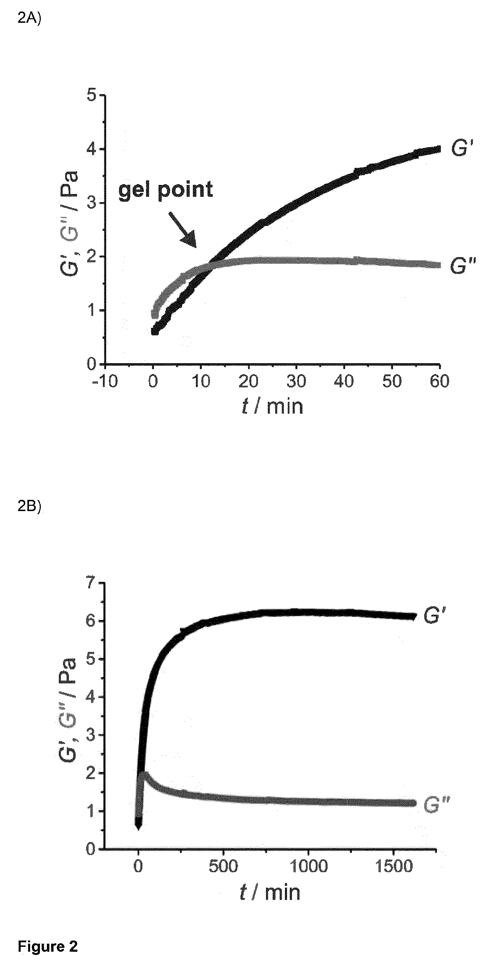

The pUC19 plasmid (2686 bp) was digested with EcoRI and Ndel restriction enzymes, resulting in two dsDNA building blocks with a size of 210 bp and 2470 bp, respectively. The thus obtained dsDNA building blocks comprise ssDNA overhangs. EcoRI produces ssDNA overhangs of four nucleotides, whereas Ndel produces ssDNA overhangs of 2 nucleotides. The obtained building blocks were separated by size. Assembly of the larger building blocks of 2470 bp by hybridization and enzymatic ligation resulted in formation of DNA hydrogel after approximately 10 minutes as shown by rheological measurements (Fig. 2A). The DNA hydrogel was rheologically observed for more than 24 hours and was stable during the whole observation (Fig. 2B). Rheological measurements were performed on a Kinexus Ultra rheometer by Malvern with an amplitude of 5% of the distance between sample plate and cone plate and a frequency of 1 Hz at a temperature of 16°C. The cone plate had a diameter of 20 mm and a cone angle of 1°.

### Description of figures

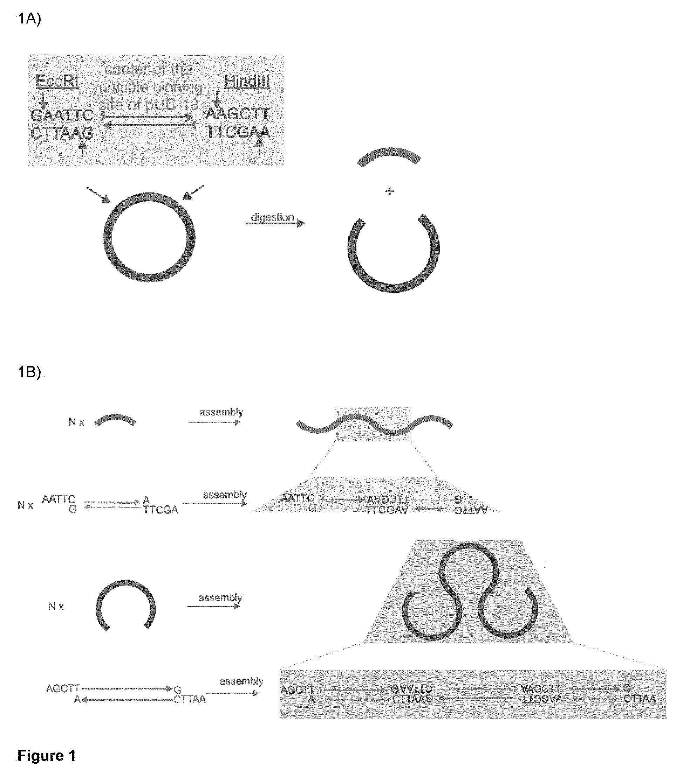

Figure 1 shows a schematic representation of an exemplary embodiment of the method of the present invention. Figure 1 A shows that digest of the pUC19 plasmid with two specific restriction enzymes results in formation of longer and shorter dsDNA building blocks. Figure 1 B shows DNA hydrogel formation by assembly of the building blocks.
Figure 2 shows the results of rheological measurements performed on assembly reactions including dsDNA building blocks of 2470 bp obtained by digest of pUC19 plasmid with EcoRI and Ndel restriction enzymes. The dsDNA building blocks comprise ssDNA overhangs. Storage modulus G' and loss modulus G" were measured with a standard rheometer. The graph shows the development of G' and G" (y-axis) over time (x-axis). Storage modulus G' is depicted in black. Loss modulus G" is depicted in grey. Figure 2A shows development of G' and G" during the first 60 minutes of the experiment. The gel point, corresponding to G'=G", is indicated. Figure 2B shows a long-time observation of G' and G" for more than 24 hours. The results show that the DNA hydrogels of the present invention are stable for a long time.

## Claims

1. Method for producing a DNA hydrogel comprising the following steps:
a) Providing basis DNA
b) Enzymatically digesting basis DNA to obtain linear dsDNA building blocks, and
c) Providing DNA hydrogel by assembly of the building blocks, wherein the assembly involves DNA hybridization and/or enzymatic ligation.

2. Method according to claim 1, wherein the basis DNA is selected from the group consisting of plasmid DNA, Herring Sperm DNA, Salmon Sperm DNA and lambda phage DNA.

3. Method according to claim 1 or 2, wherein the enzymatic digest is done with at least one restriction enzyme.

4. Method according to at least one of the preceding claims, wherein the linear dsDNA building blocks have a length of at least 10 bp.

5. Method according to at least one of the preceding claims, wherein the building blocks comprise at least one ssDNA overhang.

6. Method according to claim 5, wherein the overhang comprises at least one nucleotide.

7. Method according to at least one of the preceding claims, wherein the method further comprises the step of separating the obtained building blocks according to their length prior to assembly.

8. Method according to at least one of the preceding claims, wherein the method further comprises the step of adding non-linear dsDNA building blocks prior to assembly.

9. Method according to claim 8, wherein the non-linear dsDNA building blocks are selected from the group consisting of star-shaped dsDNA building blocks, T-shaped dsDNA building blocks, X-shaped dsDNA building blocks and Y-shaped dsDNA building blocks.

10. Method according to claim 8 or 9, wherein the non-linear dsDNA building blocks are added in an amount of at most 10 wt.-% based on the weight of the linear dsDNA building blocks.

11. Method according to at least one of the preceding claims, wherein enzymatic ligation is done with a DNA ligase.

12. Method according to claim 11, wherein the DNA ligase is selected from the group consisting of T4 DNA ligase and *E*. *coli* DNA ligase.

13. DNA hydrogel obtained by the method of at least one of the preceding claims, wherein the DNA hydrogel comprises assembled dsDNA building blocks and liquid medium.

14. DNA hydrogel according to claim 13, wherein assembled dsDNA building blocks and liquid medium constitute at least 90 wt.-% of the total weight of the DNA hydrogel.

15. Use of a DNA hydrogel according to claims 13 or 14 in applications selected from the group consisting of cell transplant therapy, bio-mineralization, tissue engineering, gene therapy, drug delivery, wound healing, cell culture, cell-free protein biosynthesis, bio-sensing, cosmetics and 3D printing.
